# EUROPEAN PATENT APPLICATION

(11) **EP 4 542 576 A1**
(43) Date of publication of application: **23.04.2025**
(21) Application number: 22958921.3
(22) Date of filing: 19.10.2022
(51) Int. Cl.: G16H 50/80, G16H 30/20, G16H 50/20, G16H 50/70, G06V 10/82, G06V 10/56, G06N 20/00

(54) **DEVICE AND METHOD FOR GENERATING INFECTION STATE INFORMATION ON BASIS OF IMAGE INFORMATION**

(30) Priority: 16.09.2022 KR 20220117347; 18.10.2022 KR 20220134365
(71) Applicant: CALTH. Inc., Seongnam-si, Gyeonggi-do 13449 (KR)
(72) Inventor: LEE, Jeong Hoon, Seongnam-si, Gyeonggi-do 13646 (KR); LEE, Ki Baek, Namyangju-si, Gyeonggi-do 12098 (KR); LEE, Seung Min, Seoul 02476 (KR); KIM, Sun Mok, Namyangju-si, Gyeonggi-do 12105 (KR); PARK, Jeong Soo, Goyang-si, Gyeonggi-do 10469 (KR); WOO, Hyo Won, Seoul 01849 (KR)
(74) Representative: Snaith, James Michael
(86) International application number: PCT/KR2022/095142
(87) International publication number: WO 2024/058319

(57) **Abstract**

According to various embodiments of the present invention, by applying a device and method for generating infection state information on the basis of image information, an input of an image of a diagnostic kit used by a user can be received, and the inputted image can be applied to a deep learning model learned using a data set obtained by augmenting diagnostic kit images in various ways, in order to generate, from the inputted image, infection state information of the user for a disease, and provide the information.

## Description

### Technical Field

The present invention relates to a device and method for generating infection state information. More specifically, the present invention relates to a device and method for generating infection state information on the basis of inputted image information. The research of the present invention is related to the 'High-sensitivity home kit for COVID-19 field screening with enhanced preprocessing (No. 2021M3E5E3080741)', a source technology development project conducted with funds from the Ministry of Science and ICT and the National Research Foundation of Korea.

### Background Art

The contents described in this section simply provide background information for the present embodiment and do not constitute prior art.

A diagnostic kit is a test tool made using a chemical reaction for the purpose of quickly and easily diagnosing some diseases. Unlike other accurate and reliable diagnostic methods, a diagnostic kit does not require special testing facilities or experts and is a test tool made to capable of quickly diagnosing infection. Diagnostic kits have the advantages of being inexpensive, being available anywhere, being easy to use, and providing diagnostic results within 15-30 minutes.

Diagnostic kits often have a problem in that the result lines that appear in a test line region and a control line region of the diagnostic kit window after the user uses the diagnostic kit are often not clearly identifiable with the naked eye, making it difficult for non-expert users to judge whether or not they are positive.

Even in cases where the result lines that appear in the test line region are difficult to identify with the naked eye, there is a need for research and development of technology that can generate and provide infection state information so that general users, i.e., non-experts, can clearly recognize whether or not they are positive.

### Prior Art Document

### Patent Document

(Patent Document 0001) Korean Patent Registration No. 10-1589673 (January 22, 2016)

### Detailed Description of Invention

### Technical Problem

An object of the present invention is to provide a device and method for generating infection state information on the basis of image information, which receives an input of an image of a diagnostic kit used by a user, applies the inputted image to a deep learning model learned using a data set obtained by augmenting diagnostic kit images in various ways, and generates and provide infection state information of the user for a disease from the inputted image.

Other unspecified purposes of the present invention can be additionally considered within the scope that can be easily inferred from the detailed description and effects thereof below.

### Solution to Problem

In order to achieve the object described above, according to one embodiment of the present invention, a computing device for generating infection state information on the basis of image information comprises a memory that stores one or more programs for generating the infection state information; and one or more processors that perform operations for generating the infection state information according to the one or more programs; and the operations performed by the processor comprise: a step of receiving an inputted image for a diagnostic kit to which a sample extracted from a test subject is applied; a step of determining a main region related to the test subject in the inputted image and determining a sub-region surrounding the main region to include it; and a step of applying the sub-region to a machine learned model to generate and output the infection state information for the test subject.

It is characterized in that the main region is a region comprising a test line whose color changes depending on the infection state of the test subject.

It is characterized in that the infection state information includes infection information indicating whether the test subject is infected or the concentration of a detection target included in the test subject predicted on the basis of the color change of the test line.

It is characterized in that the receiving of the inputted image for a diagnostic kit is receiving a plurality of inputted images including an inputted image captured at a current time point and an inputted image captured at a previous time point, and the step of generating and outputting the infection state information is generating and outputting information on the infection state trend using the concentrations of the plurality of detection targets generated on the basis of the plurality of inputted images.

It is characterized in that the step of generating and outputting the infection state information: provides a first message about the infection state at the current time point if the concentration of the detection target at the current time point has increased compared to the concentration at the previous time point, and provides a second message about the infection state at the current time point if the concentration of the detection target at the current time point has decreased compared to the concentration at the previous time point.

It is characterized in that the machine learned model is learned using an augmented data set including a plurality of training data and transformation training data that is transformed on the basis of the plurality of training data.

It is characterized in that the transformation training data includes at least one selected from the group consisting of: first transformation training data generated by blurring the training data, second transformation training data generated by adjusting the size of the training data, third transformation training data generated by distorting the training data, fourth transformation training data generated by rotating the training data, fifth transformation training data generated by adjusting the brightness of the training data, sixth transformation training data generated by changing the surrounding environment of the training data, and seventh transformation training data generated by adjusting the color temperature of the training data.

It is characterized in that the determining of the sub-region is determining to include a region corresponding to the test line and exclude a region corresponding to the control line.

It is characterized in that the step of generating and outputting the infection state information is judging whether the diagnostic kit is normal on the basis of the region corresponding to the control line, and generating and outputting information on whether the diagnostic kit is normal.

It is characterized in that the processor activates a mobile application and performs the operations through the activated mobile application.

In order to achieve the object described above, according to one embodiment of the present invention, a method for generating infection state information performed by a computing device for generating infection state information on the basis of image information may comprise: a step of receiving an inputted image for a diagnostic kit to which a sample extracted from a test subject is applied; a step of determining a main region related to the test subject in the inputted image and determining a sub-region surrounding the main region to include it; and a step of applying the sub-region to a machine learned model to generate and output the infection state information for the test subject.

It is characterized in that the main region is a region comprising a test line whose color changes depending on the infection state of the test subject.

It is characterized in that the infection state information includes infection information indicating whether the test subject is infected or the concentration of a detection target included in the test subject predicted on the basis of the color change of the test line.

It is characterized in that the determining of the sub-region is determining to include a region corresponding to the test line and exclude a region corresponding to the control line.

In order to achieve the object described above, according to one embodiment of the present invention, a computer program is stored in a computer-readable recording medium and executes any one of the methods for generating infection state information described above on a computer.

### Effects of Invention

As described above, according to one embodiment of the present invention, by applying a device and method for generating infection state information on the basis of image information, an input of an image of a diagnostic kit used by a user can be received, and the inputted image can be applied to a deep learning model learned using a data set obtained by augmenting diagnostic kit images in various ways, in order to generate, from the inputted image, infection state information of the user for a disease, and provide the information.

Even if an effect is not explicitly mentioned herein, the effect described in the following specification and its temporary effect expected by the technical features of the present invention are treated as described in the specification of the present invention.

### Brief Description of Drawings

Figure 1 is a diagram for explaining the configuration of a system comprising a device for generating infection state information on the basis of image information according to one embodiment of the present invention and a device for generating infection state information on the basis of image information.
Figure 2 is a diagram for explaining how a device for generating infection state information on the basis of image information according to one embodiment of the present invention determines a main region and a sub-region.
Figure 3 is a diagram showing a machine learned model used in a device for generating infection state information on the basis of image information according to one embodiment of the present invention.
Figure 4 is a diagram for explaining a diagnostic kit image used as a data set in a device for generating infection state information on the basis of image information according to one embodiment of the present invention.
Figure 5 is a diagram for explaining a deep learning model and data set augmentation used in a device for generating infection state information on the basis of image information according to one embodiment of the present invention.
Figure 6 is a diagram showing data comparing the performance of a device for generating infection state information on the basis of image information according to one embodiment of the present invention with the identification ability of experts and the general public as a result of using a diagnostic kit.
Figure 7 is a diagram showing data comparing the performance of a device for generating infection state information on the basis of image information according to one embodiment of the present invention with the identification ability of experts and the general public.
Figure 8 is a diagram for explaining the performance difference according to the data set used to learn a machine learned model used in a device for generating infection state information on the basis of image information according to one embodiment of the present invention and the performance difference according to the application of the diagnostic kit in different diagnostic methods.
Figure 9 is a diagram showing the positive judgment performance of a device generating infection state information on the basis of image information according to one embodiment of the present invention.
Figure 10 is a diagram showing the performance for predicting the detection target concentration according to the concentration of a sample of a device for generating infection state information on the basis of image information according to one embodiment of the present invention and the results of predicting the concentration of a detection target acquired from the inputted images acquired at time intervals.
Figure 11 is a diagram comparing the performance for predicting the concentration of a detection target of a device for generating infection state information on the basis of image information according to one embodiment of the present invention with the ability of the general public and experts to predict the concentration of a detection target.
Figure 12 is an exemplary diagram for a case where a device for generating infection state information on the basis of image information according to one embodiment of the present invention is applied to a mobile device.
Figure 13 is a diagram showing an exemplary result screen displayed to a user by a device for generating infection state information on the basis of image information according to one embodiment of the present invention.
Figure 14 is a flow chart for explaining a method for generating infection state information according to one embodiment of the present invention.
Figures 15 to 16 are block diagrams for showing a process of a method for generating infection state information according to one embodiment of the present invention.
Figure 17 is a diagram for explaining infection state trend information provided to a user by a device for generating infection state information on the basis of image information according to one embodiment of the present invention.

### Mode for Carrying out the Invention

Hereinafter, with reference to the accompanying drawings, embodiments of the present invention will be described in detail. The advantages and features of the present invention, and the methods for achieving them will become clear with reference to the embodiments described in detail below together with the accompanying drawings. However, the present invention is not limited to the embodiments disclosed below, but can be implemented in various different forms, and these embodiments are provided only to make the disclosure of the present invention complete and to fully inform a person having ordinary knowledge in the technical field to which the present invention belongs of the scope of the present invention, and the present invention is defined only by the scope of the claims. Unless otherwise defined, all terms (including technical and scientific terms) used in the present specification may be used in a meaning that can be commonly understood by a person having ordinary knowledge in the technical field to which the present invention belongs. In addition, terms defined in a commonly used dictionary shall not be interpreted ideally or excessively unless explicitly specifically defined.

The terms used in the present application are used only to describe specific embodiments and are not intended to limit the present invention. The singular expression includes the plural expression unless the context clearly indicates otherwise. In the present application, the terms such as "has," "can have," "includes," or "can include" are intended to specify the presence of a feature, number, step, operation, component, part, or a combination thereof described in the specification, but should be understood to not preclude the presence or addition of one or more other features, numbers, steps, operations, components, parts, or combinations thereof. Terms including ordinal numbers such as second, first, etc. may be used to describe various components, but the components are not limited by the terms.

The terms are used only for the purpose of distinguishing one component from another component. For example, without departing from the scope of the present invention, the second component may be referred to as the first component, and similarly, the first component may also be referred to as the second component. The term "and/or" includes a combination of a plurality of related described items or any item among a plurality of related described items.

Hereinafter, with reference to the accompanying drawings, various embodiments of a device and method for generating infection state information on the basis of image information according to the present invention will be described in detail.

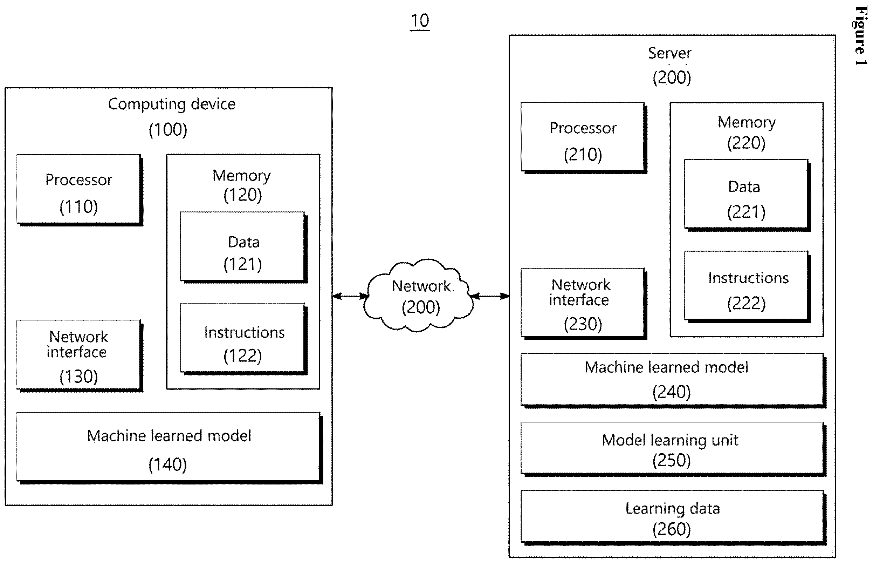

Figure 1 is a diagram for explaining the configuration of an infection state information generation system comprising a device for generating infection state information on the basis of image information according to one embodiment of the present invention and a device for generating infection state information on the basis of image information.

The infection state information generation system (10) may comprise a computing device (100) and a server (200).

A device for generating infection state information on the basis of image information may be a computing device that is configured to generate infection state information on the basis of image information.

Referring to Figure 1, a computing device (100) (hereinafter referred to as a computing device) that is configured to generate infection state information on the basis of image information may comprise a processor (110), a memory (120), a network interface (130), and a machine learned model (140).

The processor (110) may control the computing device to operate as a device for generating infection state information on the basis of image information. For example, the processor (110) may execute one or more programs stored in the memory (120). The one or more programs may comprise one or more computer-executable instructions.

The memory (120) is configured to store computer-executable instructions or program codes, program data, and/or other suitable forms of information. The computer-executable instructions or program codes, program data, and/or other suitable forms of information may also be provided through the network interface (130). The program stored in the memory (120) comprises a set of instructions (122) executable by the processor (110). In one embodiment, the memory (120) may be a memory (a volatile memory such as a random access memory, a nonvolatile memory, or a suitable combination thereof), one or more magnetic disk storage devices, optical disk storage devices, flash memory devices, or other forms of storage media that can be accessed by the computing device (100) and store desired information, or a suitable combination thereof. The memory (120) may store one or more programs for generating the infection state information.

The memory (120) may store data (121) that can be accessed by one or more processors. For example, the memory (120) may generate or store data that can be acquired, received, accessed, written, or operated by the processor (110).

The memory (120) may store instructions (122) provided in the form of a set of computer-executable instructions. The instructions (122) may be stored in the form of a program and, when executed by the processor (110), may be configured to cause the computing device (100) to perform operations according to an exemplary embodiment.

The processor (110) may be interconnected with other various components of the computing device (100) via a communication bus, including a memory (120).

The computing device (100) may also comprise one or more network interfaces (130) that provide interfaces for one or more input/output devices. The network interface (130) may operate as an input/output interface and a communication interface. The network interface (130) may be connected to the communication bus. The input/output devices (not shown) may be connected to other components of the computing device (100) via the network interface (130).

The computing device (100) may store or comprise one or more machine learned models (140). For example, the machine learned model (140) may comprise or store various machine learned models, such as neural networks (e.g., deep neural networks), support vector machines, decision trees, ensemble models, k-nearest neighbor models, Bayesian networks, other types of models including linear models and/or nonlinear models.

The neural network may include feed-forward neural networks, recurrent neural networks (e.g., long short-term memory recurrent neural networks), convolutional neural networks (CNNs), and/or other types of neural networks, or combinations thereof.

Referring to Figure 1, the server (200) may comprise a processor (210), a memory (220), a network interface (230), a machine learned model (240), a model learning unit (250), and learning data (260).

The computing device (100) and the server (200) may follow a client-server relationship.

The processor (210) may control the server to operate as a server of a device for generating infection state information on the basis of image information. For example, the processor (210) may execute one or more programs stored in the memory (220). The one or more programs may comprise one or more computer-executable instructions.

The memory (220) is configured to store computer-executable instructions or program codes, program data, and/or other suitable forms of information. The computer-executable instructions or program codes, program data, and/or other suitable forms of information may also be provided through the network interface (230). The program stored in the memory (220) comprises a set of instructions (222) executable by the processor (210). In one embodiment, the memory (220) may be a memory (a volatile memory such as a random access memory, a nonvolatile memory, or a suitable combination thereof), one or more magnetic disk storage devices, optical disk storage devices, flash memory devices, or other forms of storage media that can be accessed by the server (200) and store desired information, or a suitable combination thereof.

The memory (220) may store data (221) that can be accessed by one or more processors. For example, the memory (220) may generate or store data that can be acquired, received, accessed, written, or operated by the processor (210).

The memory (220) may store instructions (222) provided in the form of a set of computer-executable instructions. The instructions (222) may be stored in the form of a program and, when executed by the processor (210), may be configured to cause the server (200) to perform operations according to an exemplary embodiment.

The processor (210) may be interconnected with other various components of the server (200) via a communication bus, including a memory (220).

The server (200) may also comprise one or more network interfaces (230) that provide interfaces for one or more input/output devices. The network interface (230) may operate as an input/output interface and a communication interface. The network interface (230) may be connected to the communication bus. The input/output devices (not shown) may be connected to other components of the server (200) via the network interface (230).

The server (200) may store or comprise one or more machine learned models (240). For example, the machine learned model (240) may comprise or store various machine learned models, such as neural networks (e.g., deep neural networks), support vector machines, decision trees, ensemble models, k-nearest neighbor models, Bayesian networks, other types of models including linear models and/or nonlinear models.

The server (200) may comprise one or more server computing devices. These server computing devices may be, for example, parallel computing architectures or combinations thereof, and may operate according to various computing architectures, including sequential computing architectures.

The model learning unit (250) may train the machine learned models (140, 240). The model learning unit (250) may utilize one or more training or learning algorithms. The model learning unit (250) may perform training using a labeled learning data set (260), but is not necessarily limited thereto, and may also perform training using an unlabeled learning data set (260).

The learning data (260) may include a learning data set required for the model learning unit (250) to learn the machine learned model (140, 240).

The server (200) may communicate with the computing device (100) via the network (300).

The network (300) may be any type of network or a combination of networks that enable communication between devices. The network (300) may include at least one of a local area network, a wide area network, the Internet, a secure network, a cellular network, a mesh network, and a peer-to-peer communication link.

The processor (110) may receive an inputted image for a diagnostic kit to which a sample extracted from a test subject is applied, determine a main region related to the test subject in the inputted image, determine a sub-region surrounding the main region to include it, and apply the sub-region to a machine learned model to generate and output the infection state information for the test subject.

Here, the test subject may include all of a human, an animal, and a plant.

The sample may be collected from the test subject, and may be saliva, spit, nasal mucus, blood, urine, and the like of the test subject, and may be applied to the diagnostic kit after being mixed with other substances, or ay be applied to the diagnostic kit as a sample itself.

The server (200) may collect and manage infection state information from the computing device (100). On the basis of the infection state information collected from the computing device (100), it may be possible for a hospital or a specialized institution to accurately analyze and manage the user's symptoms.

The server (200) may use a database (not shown) that stores and manages not only the infection state information collected from the computing device (100) but also the user's personal information and information analyzed by experts.

Not all blocks shown in Figure 1 are essential components, and in other embodiments, some blocks connected to the device (100) for generating infection state information on the basis of image information and the system (10) that comprises a device for generating infection state information on the basis of image information may be added, changed, or deleted.

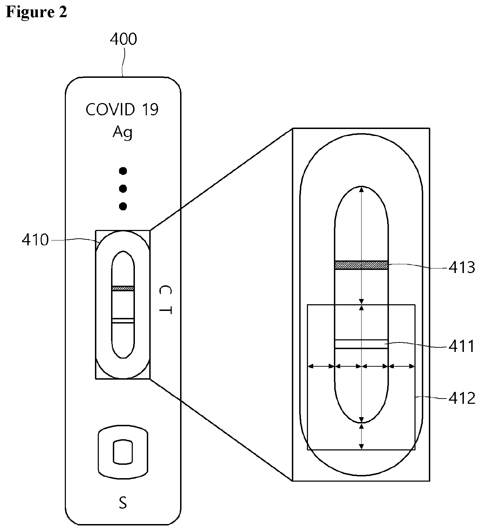

Figure 2 is a diagram for explaining how a device for generating infection state information on the basis of image information according to one embodiment of the present invention determines a main region and a sub-region.

Reference symbol 400 indicates a diagnostic kit. The diagnostic kit may be a device used to judge whether the user is infected with a disease through a test subject collected from the user or a sample mixed with the test subject. The diagnostic kit may be, for example, a diagnostic kit for COVID-19.

Reference symbol 410 indicates a window in which an indicator line (e.g., a test line, a control line) indicating a test result appears after a user uses a diagnostic kit.

The processor (110) may determine a main region related to the test subject by detecting a region corresponding to a test line (411) of a window (410) in which a test result of a diagnostic kit (400) appears.

The main region may be a region comprising a test line whose color changes depending on the infection state of the test subject.

The processor (110) may determine a sub-region by determining to include a region corresponding to the test line (411) and exclude a region corresponding to the control line (413).

The processor (110) may determine a sub-region to include a region corresponding to the test line (413), a portion of a region corresponding to the window (410) excluding a region corresponding to the control line (413), and at least a portion of a region outside the region corresponding to the window (410) and corresponding to the diagnostic kit (400).

For example, the processor (110) may determine a region corresponding to half of a region corresponding to the window (410) where the test line (413) is located as a sub-region, and may also determine a sub-region to further include a region corresponding to the diagnostic kit (400).

The processor (110) may judge whether the test subject is recognized to be positive on the basis of the degree of darkness of the test line (411). The processor (110) may generate and output infection state information including information on whether the test subject is positive.

Infection state information may include infection information indicating whether the test subject is infected, or the concentration of the detection target included in the test subject predicted on the basis of the color change of the test line (411). The color change may be determined on the basis of the degree of darkness of the test line.

The processor (110) may generate concentration information of the detection target included in the test subject predicted on the basis of the degree of darkness of the test line (411).

The degree of darkness may be divided into predetermined standard stages on the basis of preset criteria. For example, the degree of darkness may be divided into a total of 11 stages from stage 0 to stage 10 in order of lowest to highest degree of darkness. The processor (110) may judge that the test line (411) is positive if the degree of darkness is equal to or higher than a predetermined threshold stage (for example, stage 3). The degree of darkness may be chroma.

The detection target may be various types of viruses or bacteria, such as HIV (Human Immunodeficiency Virus), COVID-19 virus, and influenza virus.

The processor (110) may generate and output infection state information including the concentration information of the generated detection target.

The processor (110) may predict the concentration of the detection target and generate the concentration information of the detection target by matching the degree of darkness with the concentration of the detection target.

For example, if the processor (110) judges that the degree of darkness of the test line (411) is stage 1, it may predict that the concentration of the detection target is 10 ng/ml, and if it judges that the degree of darkness of the test line (411) is stage 10, it may predict that the concentration of the detection target is 100 ng/ml. In other words, it may have a concentration interval of 10 ng/ml for each unit stage interval.

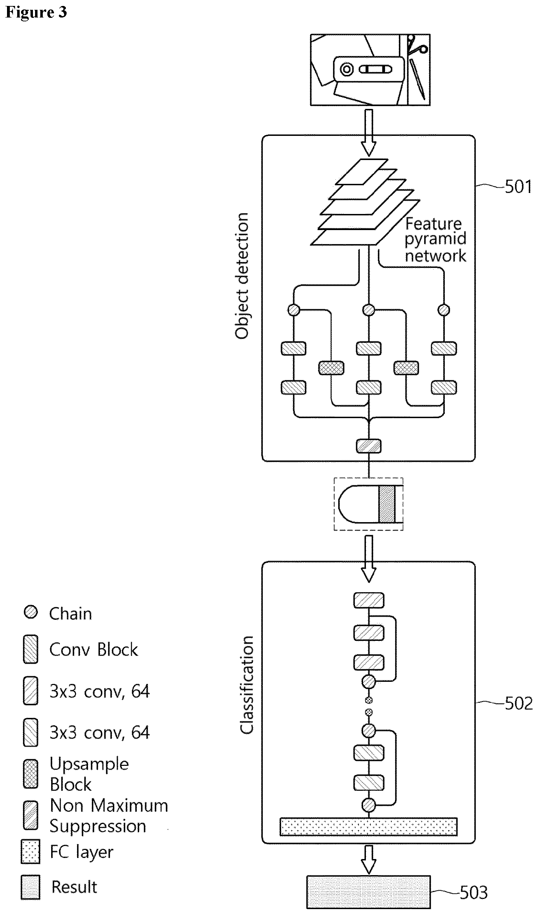

Figure 3 is a diagram showing a machine learned model used in a device for generating infection state information on the basis of image information according to one embodiment of the present invention.

Referring to Figure 3, the method for generating infection state information according to the present invention may include an object detection process for detecting a diagnostic kit from an inputted image and a process for classifying the presence or absence of a detection target (i.e., whether positive or not) or the degree of darkness of a test line from a main region or a sub-region portion determined from an inputted image.

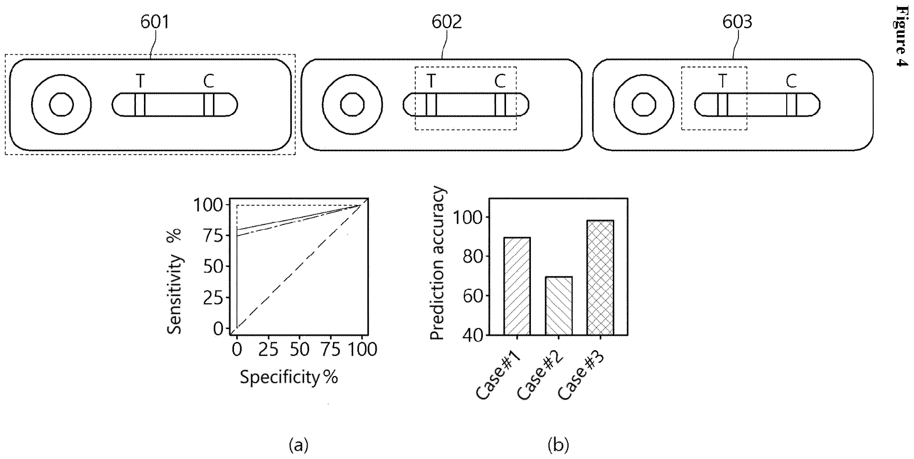

Figure 4 is a diagram for explaining a diagnostic kit image used as a data set in a device for generating infection state information on the basis of image information according to one embodiment of the present invention.

Figure 4 may be a concept corresponding to reference symbol 501 of Figure 3.

Reference symbol 601 of Figure 4 indicates a case where the entire diagnostic kit is cropped from an inputted image.

Reference symbol 602 of Figure 4 indicates that the inputted image is cropped to include both the test line and the control line.

Reference symbol 603 of Figure 4 indicates that the inputted image is cropped to include both the test line and not the control line.

(a) and (b) of Figure 4 are graph data showing that in the case corresponding to reference symbol 603, it is more accurate for the computing device (100) to judge whether it is infected or not than in the case corresponding to reference symbol 602, and in the case corresponding to reference symbol 602, it is more accurate for the computing device (100) to judge whether or not it is infected than in the case corresponding to reference symbol 601.

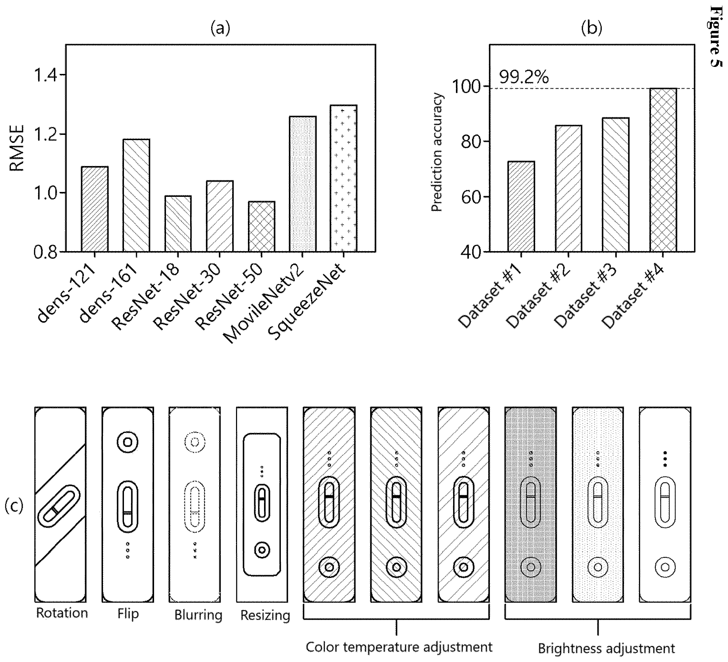

Figure 5 is a diagram for explaining a deep learning model and data set augmentation used in a device for generating infection state information on the basis of image information according to one embodiment of the present invention.

(a) of Figure 5 is a diagram showing RMSD (Root Mean Square Deviation) according to CNN models.

(b) of Figure 5 is a diagram showing the diagnostic accuracy of the test subject of the computing device (100) according to the type of data set.

Dataset #1 is a case where the diagnostic kit images captured under a single environment and a single light are the data set, Dataset #2 is a case where the dataset #1 is augmented in the manner of Figure 5 (c), Dataset #3 is a case where the diagnostic kit images captured under various lights are additionally included in Dataset #2, and Dataset #4 is a case where the diagnostic kit images captured under various lights and in various environments are additionally included in Dataset #3.

That is, it can be confirmed that the performance of the computing device (100) increases as the types of images included in the data set become more diverse.

In the present invention, the machine learned model used by the computing device (100) may be learned using an augmented data set including a plurality of training data and transformation training data transformed on the basis of the plurality of training data. In the present invention, the machine learned model used by the computing device (100) may be a deep learning model.

Referring to (c) of Figure 5, the learning data (260) may include an augmented data set by using at least one of a blurring method, a resizing method, a distorting method, a rotating method, a brightness adjustment method, a different surrounding environment method, and a different color temperature method of the diagnostic kit images before and after use, which are learning images, to increase the number of data sets.

That is, the transformation training data may include at least one selected from the group consisting of first transformation training data generated by blurring the training data, second transformation training data generated by adjusting the size of the training data, third transformation training data generated by distorting the training data, fourth transformation training data generated by rotating the training data, fifth transformation training data generated by adjusting the brightness of the training data, sixth transformation training data generated by changing the surrounding environment of the training data, and seventh transformation training data generated by adjusting the color temperature of the training data.

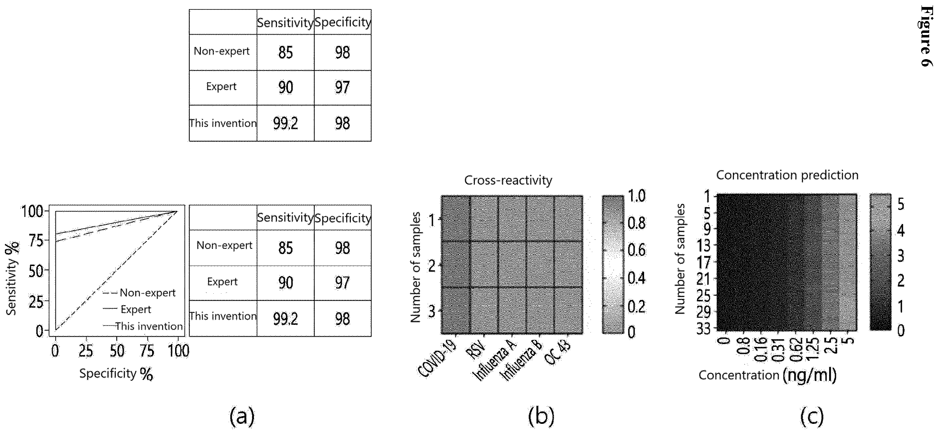

Figure 6 is a diagram showing data comparing the performance of a device for generating infection state information on the basis of image information according to one embodiment of the present invention with the identification ability of experts and the general public as a result of using a diagnostic kit.

(a) of Figure 6 is a diagram showing a receiver operating characteristic (ROC) curve showing the prediction performance of a non-expert, expert, and computing device (100) for the test result judgment performance after a user uses a diagnostic kit.

(b) of Figure 6 is a diagram showing experimental data for confirming cross-reactivity.

(c) of Figure 6 is data showing the performance for predicting the concentration of a detection target. It can be confirmed that the concentration prediction performance is excellent through the fact that the color corresponding to the actual concentration and the predicted color are similar.

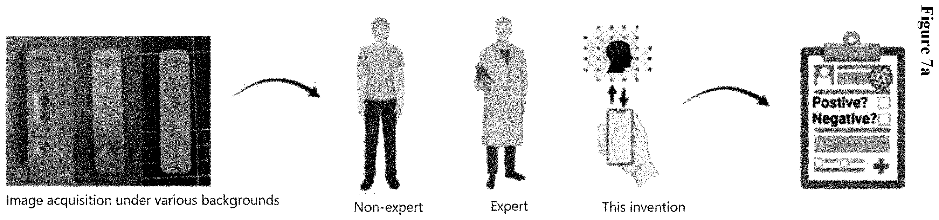

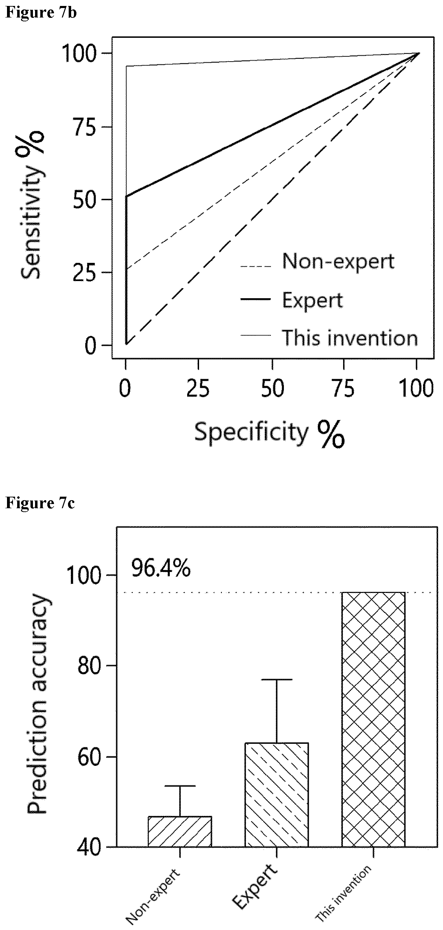

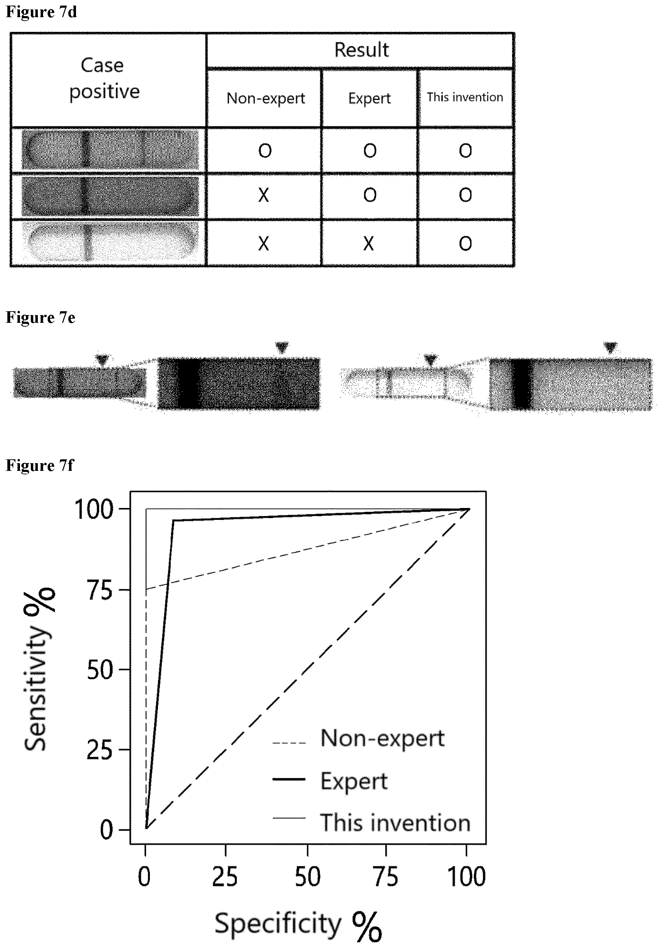

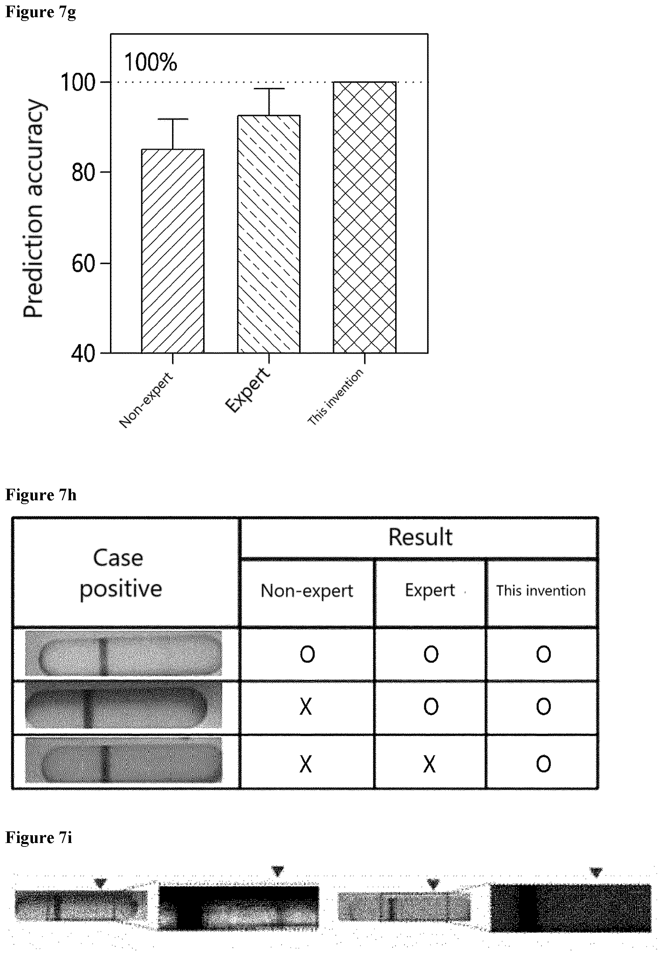

Figure 7 is a diagram showing data comparing the performance of a device for generating infection state information on the basis of image information according to one embodiment of the present invention with the identification ability of experts and the general public.

(b) of Figure 7 to (e) of Figure 7 are diagrams showing the results of a blind test on the ability of a computing device (100), an expert, and a non-expert to recognize the positivity of a diagnostic kit used in the nasal method.

In the case of non-experts, the accuracy was 46.4%, and the dev. was 7.1. In the case of experts, the accuracy was 63.2%, and the dev. was 13.7. In the case of the present invention, the accuracy was 97.5%.

(d) of Figure 7 is a diagram showing the actual image matched by the general public in the blind test, the image matched only by experts and the computing device (100), and the image matched only by the computing device (100).

(e) of Figure 7 is a diagram showing the image processing of the second image that was not identified by the general public, non-experts, and the third image that was not identified by the non-experts and the experts, among the images of (d) of Figure 7.

In the case of the second image that was not identified by the general public, non-experts, but was identified by the experts and the computing device (100), a weak color development appeared that was not easy to identify with the naked eye, but was enough for the experts to identify, and the test line was faintly identified as a result of the image processing by adjusting the brightness and contrast.

In the case of the third image that was not identified by the non-experts and the experts, and was identified by the computing device (100), it can be confirmed that there is no difference even if the image processing is performed because it is difficult to detect with the naked eye. In other words, the computing device (100) may recognize the test line that is too dark to be detected with the naked eye and may judge that the test subject is positive.

(f) of Figure 7 to (i) of Figure 7 are diagrams showing the results of a blind test on the ability of a computing device (100), an expert, and a non-expert to recognize the positivity of a diagnostic kit used in the saliva method.

In the case of non-experts, the accuracy was 85%, and the dev. was 7. In the case of experts, the accuracy was 92.5%, and the dev. was 5.6. In the case of the present invention, the accuracy was 100%.

(h) of Figure 7 is a diagram showing the actual image matched by the general public in the blind test, the image matched only by experts and the computing device (100), and the image matched only by the computing device (100).

(i) of Figure 7 is a diagram showing the image processing of the second image that was not identified by the general public, non-experts, and the third image that was not identified by the non-experts and the experts, among the images of (h) of Figure 7.

In the case of the second image that was not identified by the general public, non-experts, but was identified by the experts and the computing device (100), a weak color development appeared that was not easy to identify with the naked eye, but was enough for the experts to identify, and the test line was faintly identified as a result of the image processing by adjusting the brightness and contrast.

In the case of the third image that was not identified by the non-experts and the experts, and was identified by the computing device (100), it can be confirmed that there is no difference even if the image processing is performed because it is difficult to detect with the naked eye. In other words, the computing device (100) may recognize the test line that is too dark to be detected with the naked eye and may judge that the test subject is positive.

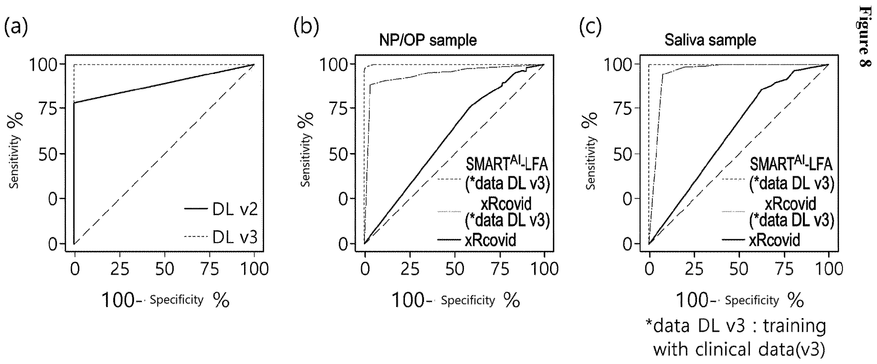

Figure 8 is a diagram for explaining the performance difference according to the data set used to learn a machine learned model used in a device for generating infection state information on the basis of image information according to one embodiment of the present invention and the performance difference according to the application of the diagnostic kit in different diagnostic methods.

(a) of Figure 8 is a graph showing that the performance is improved when a clinical sample is added (DL..v3) to the image (DL..v2) of a kit in which a target substance is diluted in a standard sample (phosphate buffered saline). As shown, it can be seen that the performance of the computing device (100) becomes better when there is more data.

(b) to (c) of Figure 8 are graphs comparing the performance of the present invention and the conventional technology in diagnosing whether or not a diagnosis is positive from an image of a diagnostic kit using the nasal method and the saliva method, respectively.

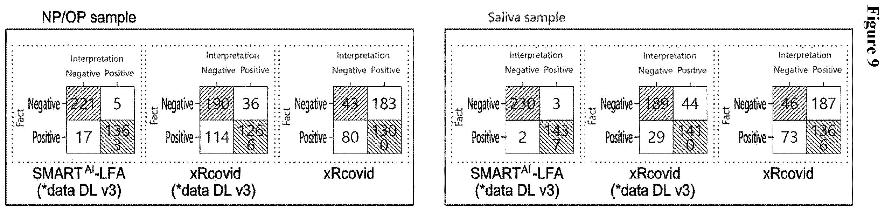

Figure 9 is a diagram showing the positive judgment performance of a device generating infection state information on the basis of image information according to one embodiment of the present invention.

Figure 9 is a confusion matrix showing how accurately the computing device (100) according to the present invention judges whether or not a diagnosis is positive or negative on the basis of a sample used in a cell membrane.

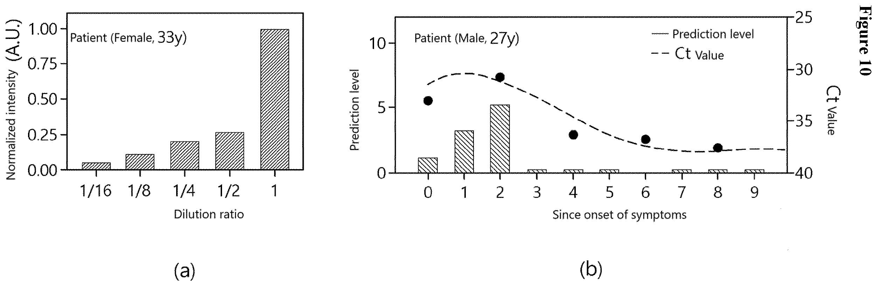

Figure 10 is a diagram showing the performance for predicting the detection target concentration according to the concentration of a sample of a device for generating infection state information on the basis of image information according to one embodiment of the present invention and the results of predicting the concentration of a detection target acquired from the inputted images acquired at time intervals.

The receiving of the inputted image for a diagnostic kit by the processor (110) may be receiving a plurality of inputted images including an inputted image captured at a current time point and an inputted image captured at a previous time point.

It is common for a user to perform self-diagnosis a plurality of times using a diagnostic kit in order to confirm his/her own diagnosis results. For example, a user may perform self-diagnosis twice a day for 5 to 9 consecutive days using a diagnostic kit. In response, the computing device (100) may provide information on the infection state trend.

The infection state information may further comprise information on the infection state trend.

The processor (110) may generate the concentration information of the detection target included in the test subject predicted on the basis of the degree of darkness of the test line (411).

The processor (110) may predict the concentration of the detection target and generate the concentration information of the detection target by matching the degree of darkness with the concentration of the detection target.

For example, if the processor (110) judges that the degree of darkness of the test line (411) is stage 1, it may predict that the concentration of the detection target is 10 ng/ml, and if it judges that the degree of darkness of the test line (411) is stage 10, it may predict that the concentration of the detection target is 100 ng/ml. In other words, it may have a concentration interval of 10 ng/ml for each unit stage interval. In addition, if the processor (110) judges that the degree of darkness of the test line (411) is between stage 5 and stage 6 (for example, stage 5.6), it is natural that it may predict that the concentration of the detection target is between 50 ng/ml and 60 ng/ml (for example, 56 ng/ml).

The processor (110) may receive a plurality of inputted images captured at different time points. The processor (110) may generate and output information on the infection state trend using the concentration information of the plurality of detection targets generated on the basis of the plurality of inputted images.

The processor (110) may generate and output infection state information by providing a first message about the infection state at the current time point if the concentration of the detection target at the current time point has increased compared to the concentration at the previous time point, and by providing a second message about the infection state at the current time point if the concentration of the detection target at the current time point has decreased compared to the concentration at the previous time point.

The first message may include a message indicating that the test subject is in the infectious period. The first message may further include a message indicating that a PCR test is recommended because the test subject is in the infectious period.

The second message may include a message indicating that the test subject is in the recovery period.

The processor (110) may set the date of receiving the first inputted image as the first day, and may predict the concentration of the detection target on the basis of the inputted images that are inputted thereafter, and may generate and output infection state trend information corresponding to the input time or date of the inputted images.

(a) of Figure 10 is a diagram showing that the predicted concentration gradually decreases as the computing device (100) dilutes a sample for a patient with phosphate buffered saline (PBS).

(b) of Figure 10 is a graph showing the infection state trend information generated by the computing device (100) and the PCR test result trend information. Referring to (b) of Figure 10, it can be confirmed that the infection state trend information generated by the computing device (100) and the PCR test result show substantially similar trends.

Referring to (b) of Figure 10, it can be confirmed that the concentration of the detection target included in the body gradually decreases over time after the user is infected.

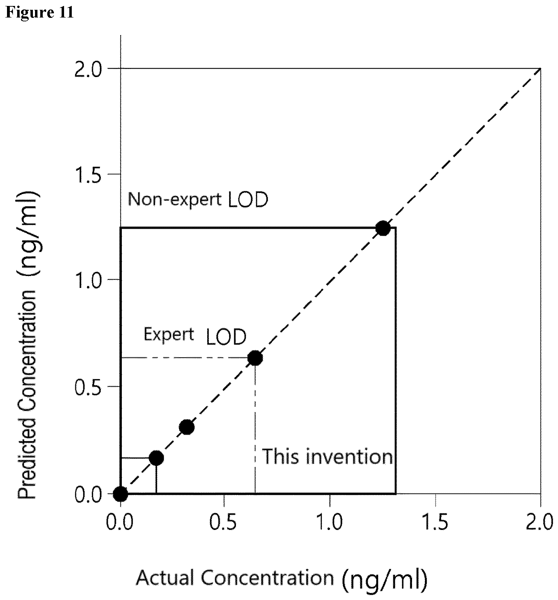

Figure 11 is a diagram comparing the performance for predicting the concentration of a detection target of a device for generating infection state information on the basis of image information according to one embodiment of the present invention with the ability of the general public and experts to predict the concentration of a detection target.

Figure 11 is a graph comparing the concentration of the test subject sample applied to the diagnostic kit and the concentration predicted from the image of the diagnostic kit by non-experts, experts, and the computing device (100). The closer it is to the Y=X graph, the better the prediction may be judged.

Referring to Figure 11, the lowest concentration (LOD) that the general public can judge as positive with the naked eye is 1.25 ng/ml, the lowest concentration that an expert can judge as positive with the naked eye is 0.62 ng/ml, and the computing device (100) according to the present invention using deep learning was able to judge as positive up to 0.15 ng/ml.

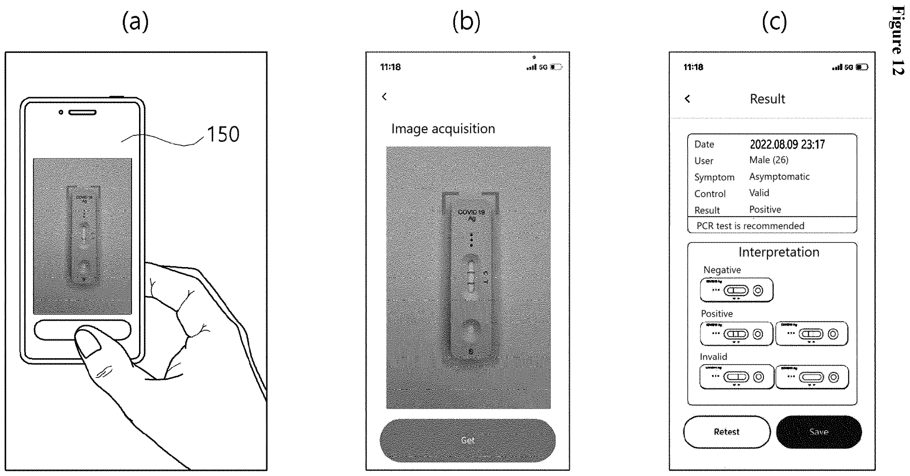

Figure 12 is an exemplary diagram for a case where a device for generating infection state information on the basis of image information according to one embodiment of the present invention is applied to a mobile device.

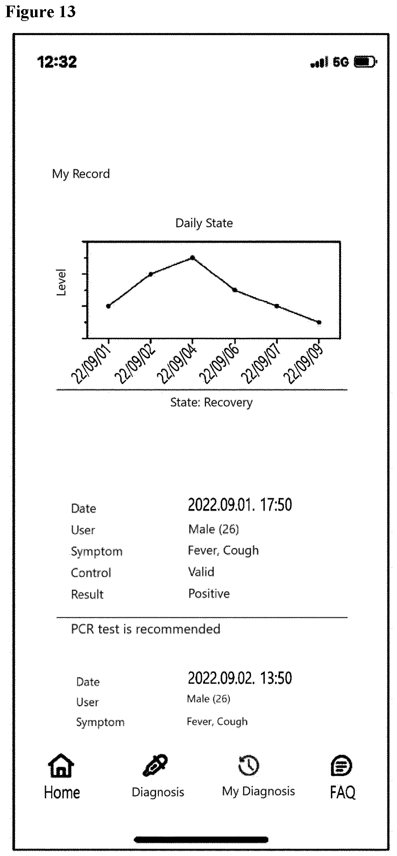

Figure 13 is a diagram showing an exemplary result screen displayed to a user by a device for generating infection state information on the basis of image information according to one embodiment of the present invention.

The computing device (100) according to the present invention is an electronic device for analyzing an inputted image to judge whether a test subject applied to a diagnostic kit is positive, and may include various types of portable terminal devices such as smartphones, tablet PCs, and desktop PCs. Accordingly, the processor (110) may activate a mobile application and performing the operations through the activated mobile application.

Referring to (a) of Figure 12, the user may capture an image of a diagnostic kit applied to a test subject using the computing device (100). As shown in (a) of Figure 12, the user may directly acquire the diagnostic kit image using the computing device (100) equipped with a camera, but the present invention is not limited thereto, and the computing device (100) may also receive the diagnostic kit image acquired using an external device.

Referring to (b) of Figure 12, the user may activate the mobile application for performing operations according to the present invention through the computing device (100). The mobile application may operate by activating an inputted image stored in the computing device (100) in response to the user's operation, and the user may also capture the diagnostic kit through the activated mobile application.

The user inputs information such as his/her symptoms (for example, fever, cough, runny nose, phlegm, etc.), gender, age, height, body weight, and time of use of the diagnostic kit into the activated mobile application, and the computing device (100) may generate infection state information including various types of information inputted by the user.

Referring to (c) of Figure 12, the computing device (100) may generate infection state information on the basis of the inputted image activated through the mobile application, and the generated infection state information may be displayed to the user through the display unit (150) of the computing device (100). The computing device (100) may display the infection state information to the user through the display unit (150) included in the computing device (100), and may also transmit the infection state information to an external device so that the external device can display the infection state information to the user.

The processor (110) may judge whether the diagnostic kit is normal on the basis of the region corresponding to the control line from the inputted image, and may generate and output information on whether the diagnostic kit is normal. The information on whether the diagnostic kit is normal may be displayed together with the infection state information.

The processor (110) may judge that the diagnostic kit is faulty if neither the control line nor the test line is recognized in the diagnostic kit included in the inputted image, or if the test line appears but the control line is not recognized.

Referring to Figure 13, the computing device (100) may generate information on the infection state trend as described in Figure 10, and may display the infection state information including the generated information on the infection state trend to the user. The information on the infection state trend may be provided as shown in Figure 13, with the X-axis representing information on the date and the Y-axis representing information on the concentration of the detection target.

In this case, the processor (110) may judge that the period from September 1, 2022 to September 4, 2022 is an infectious period, and that the period from September 4, 2022 to September 9, 2022 is a recovery period, and may display the judged information to the user by including it in the infection state information.

As shown in Figure 13, the computing device (100) may display a message recommending a PCR (Polymerase Chain Reaction) test if the user is judged to be positive through a diagnostic kit.

The computing device (100) may also transmit the generated infection state information to a specialized medical institution.

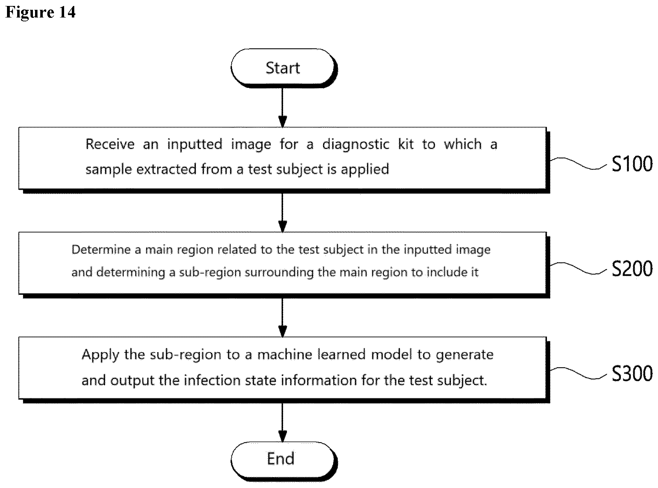

Figure 14 is a flow chart for explaining a method for generating infection state information according to one embodiment of the present invention.

The method for generating infection state information may be performed by a computing device for generating infection state information on the basis of image information.

At step S100, the processor may perform a step of receiving an inputted image for a diagnostic kit to which a sample extracted from a test subject is applied.

At step S200, the processor may perform a step of determining a main region related to the test subject in the inputted image and determining a sub-region surrounding the main region to include it; and

At step S300, the processor may perform a step of applying the sub-region to a machine learned model to generate and output the infection state information for the test subject.

Although each process is described as being executed sequentially in Figure 14, this is merely an example, and those of ordinary skill in the art will be able to apply various modifications and variations, such as changing the order described in Figure 14, executing one or more processes in parallel, or adding other processes, without departing from the essential characteristics of the embodiments of the present invention.

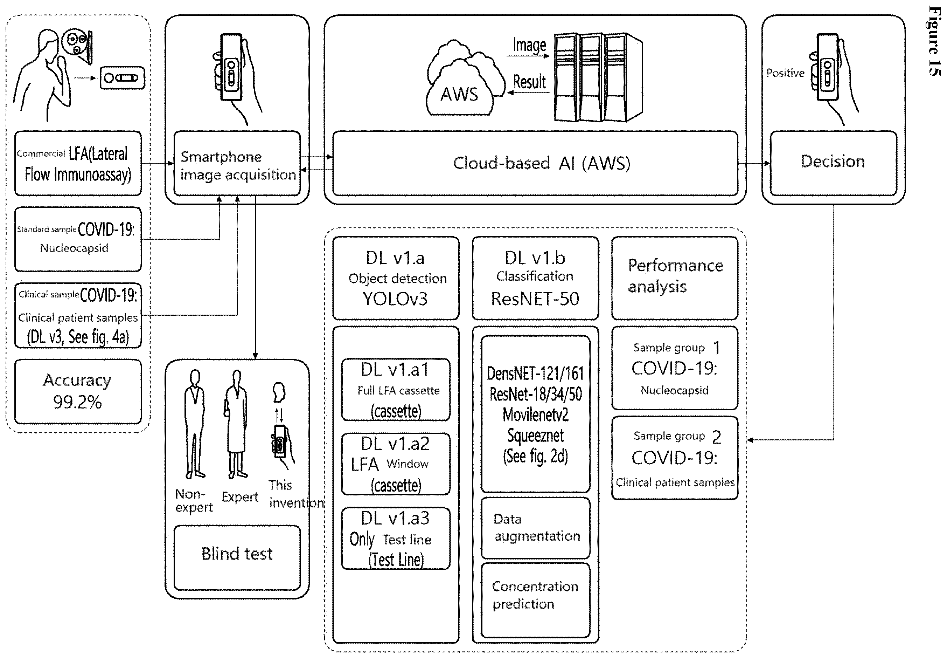

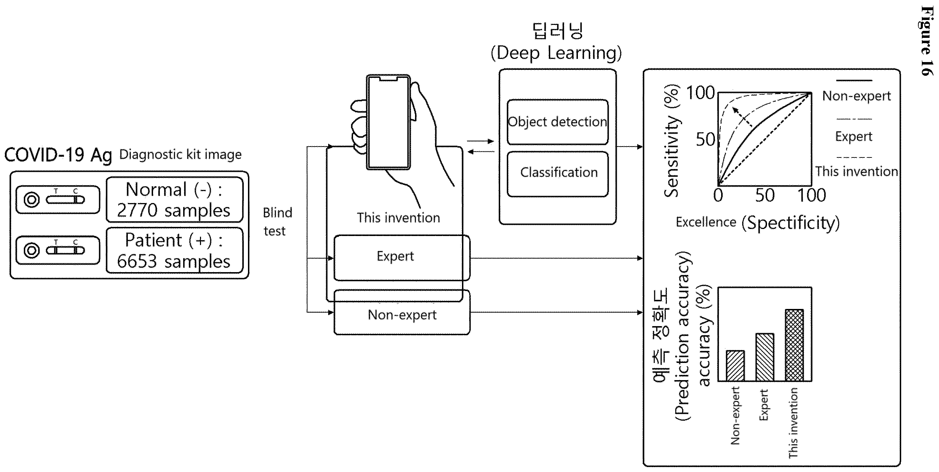

Figures 15 to 16 are block diagrams for showing a process of a method for generating infection state information according to one embodiment of the present invention.

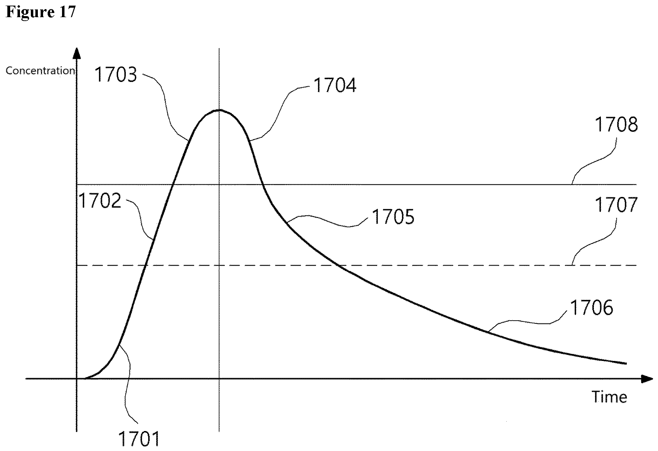

Figure 17 is a diagram for explaining infection state trend information provided to a user by a device for generating infection state information on the basis of image information according to one embodiment of the present invention.

As described in Figure 10, the computing device (100) may generate information on the infection state trend and may display the infection state information including the generated information on the infection state trend to the user.

The processor (110) may generate and output infection state information by providing a first message about the infection state at the current time point if the concentration of the detection target at the current time point has increased compared to the concentration at the previous time point, and by providing a second message about the infection state at the current time point if the concentration of the detection target at the current time point has decreased compared to the concentration at the previous time point.

The information on the infection state trend may include the first message or the second message.

Figure 17 is reference graph data showing the concentration of a virus detected over time after being infected with a disease (for example, coronavirus).

The reference graph data on the virus concentration collected from the infected person over time may be statistical data, and may be data generated on the basis of previously known data and stored in advance in the computing device (100).

The processor may determine which infection section the test subject corresponds to by comparing the concentration at the current time point and the concentration at the previous time point with the reference graph data to provide the user with information on the infection state trend of the test subject.

The infection section of the test subject may include a preliminary section (1701), a first infection section (1702), a second infection section (1703), a third infection section (1704), a fourth infection section (1705), and a state improvement section (1706).

For example, the processor (110) may judge that the test target corresponds to the preliminary section (1701) if the concentration at the current time point is higher than the concentration at the previous time point and the concentration at the current time point is lower than or equal to the first threshold concentration (1707). In this case, the first message may include a message indicating that the test subject is not yet infected, but is expected to be infected.

The processor (110) may judge that the test subject is in the first infection section (1702) if the concentration at the current time point is higher than the concentration at the previous time point, and the concentration at the current time point is higher than or equal to the first threshold concentration (1707) and is lower than or equal to the second threshold concentration (1708). In this case, the first message may include a message indicating that the test subject is infected and is expected to be in the initial infection state.

The processor (110) may judge that the test subject is in the second infection section (1703) if the concentration at the current time point is higher than the concentration at the previous time point, and the concentration at the current time point is higher than or equal to the second threshold concentration (1708). In this case, the first message may include a message indicating that the test subject is highly likely to be infected and may require treatment from a specialized institution.

The processor (110) may judge that the test subject is in the third infection section (1704) if the concentration at the current time point is lower than the concentration at the previous time point and the concentration at the current time point is higher than or equal to the second threshold concentration (1708). In this case, the second message may include a message indicating that the test subject is highly likely to be infected, but that the state is improving.

The processor (110) may judge that the test subject is in the fourth infection section (1705) if the concentration at the current time point is lower than the concentration at the previous time point, and the concentration at the current time point is higher than or equal to the first threshold concentration (1707) and is lower than or equal to the second threshold concentration (1708). In this case, the second message may include a message indicating that the test subject is expected to be in the late infection state.

The processor (110) can judge that the test subject is in the state improvement section (1706) if the concentration at the current time point is lower than the concentration at the previous time point and the concentration at the current time point is lower than or equal to the first threshold concentration (1707). In this case, the second message may include a message indicating that the test subject is not infected, the infection state has improved, and daily life is possible.

Here, the first threshold concentration (1707) may be a reference concentration for judging whether the test subject is positive. In other words, the processor (110) may judge that the test subject is positive if the concentration of the detection target collected from the test subject is higher than or equal to the first threshold concentration (1707).

The second threshold concentration (1708) may be a reference concentration for judging whether the test subject is in a severe condition beyond the condition diagnosed to be positive. In other words, the processor (110) may judge that the test subject is in a severe condition if the concentration of the detection target collected from the test subject is higher than or equal to the second threshold concentration (1708).

The first threshold concentration (1707) and the second threshold concentration (1708) may be reference values determined in advance on the basis of statistical data.

The processor (110) may determine the infection section of the test subject using the concentration at the current time point and the concentration at the previous time point, and may also determine the infection section of the test subject by further considering the slope value calculated on the basis of the time interval between the current time point and the previous time point and the difference between the concentration at the current time point and the concentration value at the previous time point.

The processor (110) may predict the user's infection time point using at least one of the determined infection section of the test subject, the reference graph data, and the slope value on the basis of the concentration at the current time point and the concentration at the previous time point. The infection state information may include the predicted infection time point.

The present application also provides a computer storage medium. The computer storage medium stores program instructions, and when the program instructions are executed by the processor, the method for generating infection state information as described above is realized.

The computer storage medium according to one embodiment of the present invention may be a U disk, an SD card, a PD optical drive, a mobile hard disk, a large-capacity floppy drive, a flash memory, a multimedia memory card, a server, etc., but is not necessarily limited thereto.

Even though all the components constituting the embodiments of the present invention described above are described as being combined or operating in combination as one, the present invention is not necessarily limited to these embodiments. That is, within the scope of the purpose of the present invention, all of the components may be selectively combined one or more times and operated. In addition, although each of the components may be implemented as an independent piece of hardware, some or all of the components may be selectively combined to implement a computer program having program modules that perform some or all of the functions of a single piece of hardware or a plurality of pieces of hardware. In addition, such a computer program may be stored in a computer readable medium such as a USB memory, a CD disk, a flash memory, etc., and read and executed by a computer, thereby implementing one embodiment of the present invention. The recording medium of the computer program may include a magnetic recording medium, an optical recording medium, and the like.

The above description is merely an example of the technical idea of the present invention, and a person having ordinary knowledge in the technical field to which the present invention belongs may make various modifications, changes, and substitutions within a scope that does not deviate from the essential characteristics of the present invention. Therefore, the embodiments disclosed in the present invention and the accompanying drawings are not intended to limit the technical idea of the present invention, but are intended to explain it, and the scope of the technical idea of the present invention is not limited by these embodiments and the accompanying drawings. The protection scope of the present invention should be interpreted by the claims below, and all technical ideas within the equivalent scope should be interpreted as being included in the scope of the rights of the present invention.

### [Description of symbols]

100: Computing device
110: Processor
120: Memory
130: Network interface
140: Machine learned model
200: Server
300: Network

## Claims

1. A computing device for generating infection state information on the basis of image information,
the computing device comprises a memory that stores one or more programs for generating the infection state information; and one or more processors that perform operations for generating the infection state information according to the one or more programs; and the operations performed by the processor comprise:
a step of receiving an inputted image for a diagnostic kit to which a sample extracted from a test subject is applied;
a step of determining a main region related to the test subject in the inputted image and determining a sub-region surrounding the main region to include it; and
a step of applying the sub-region to a machine learned model to generate and output the infection state information for the test subject.

2. The computing device according to claim 1, wherein the main region is a region comprising a test line whose color changes depending on the infection state of the test subject.

3. The computing device according to claim 2, wherein the infection state information includes infection information indicating whether the test subject is infected or the concentration of a detection target included in the test subject predicted on the basis of the color change of the test line.

4. The computing device according to claim 3, wherein
the receiving of the inputted image for a diagnostic kit is receiving a plurality of inputted images including an inputted image captured at a current time point and an inputted image captured at a previous time point, and
the step of generating and outputting the infection state information is generating and outputting information on the infection state trend using the concentrations of the plurality of detection targets generated on the basis of the plurality of inputted images.

5. The computing device according to claim 4, wherein the step of generating and outputting the infection state information:
provides a first message about the infection state at the current time point if the concentration of the detection target at the current time point has increased compared to the concentration at the previous time point, and
provides a second message about the infection state at the current time point if the concentration of the detection target at the current time point has decreased compared to the concentration at the previous time point.

6. The computing device according to claim 1, wherein the machine learned model is learned using an augmented data set including a plurality of training data and transformation training data that is transformed on the basis of the plurality of training data.

7. The computing device according to claim 6, wherein the transformation training data includes at least one selected from the group consisting of:
first transformation training data generated by blurring the training data,
second transformation training data generated by adjusting the size of the training data,
third transformation training data generated by distorting the training data,
fourth transformation training data generated by rotating the training data,
fifth transformation training data generated by adjusting the brightness of the training data,
sixth transformation training data generated by changing the surrounding environment of the training data, and
seventh transformation training data generated by adjusting the color temperature of the training data.

8. The computing device according to claim 2, wherein the determining of the sub-region is determining to include a region corresponding to the test line and exclude a region corresponding to the control line.

9. The computing device according to claim 8, wherein the step of generating and outputting the infection state information is judging whether the diagnostic kit is normal on the basis of the region corresponding to the control line, and generating and outputting information on whether the diagnostic kit is normal.

10. The computing device according to claim 1, wherein the processor activates a mobile application and performs the operations through the activated mobile application.

11. A method for generating infection state information performed by a computing device for generating infection state information on the basis of image information, the method comprising:
a step of receiving an inputted image for a diagnostic kit to which a sample extracted from a test subject is applied;
a step of determining a main region related to the test subject in the inputted image and determining a sub-region surrounding the main region to include it; and
a step of applying the sub-region to a machine learned model to generate and output the infection state information for the test subject.

12. The method for generating infection state information according to claim 11, wherein the main region is a region comprising a test line whose color changes depending on the infection state of the test subject.

13. The method for generating infection state information according to claim 12, wherein the infection state information includes infection information indicating whether the test subject is infected or the concentration of a detection target included in the test subject predicted on the basis of the color change of the test line.

14. The method for generating infection state information according to claim 12, wherein the determining of the sub-region is determining to include a region corresponding to the test line and exclude a region corresponding to the control line.

15. A computer program stored in a computer-readable recording medium for executing the method for generating infection state information according to any one of claims 11 to 14 on a computer.
